# EUROPEAN PATENT SPECIFICATION

(11) **EP 1 509 215 B1**
(45) Date of publication and mention of the grant of the patent: **02.11.2006**
(21) Application number: 03725238.4
(22) Date of filing: 14.05.2003
(51) Int. Cl.: A61K 31/085, A61P 15/02

(54) **TREATMENT OR PREVENTION OF UROGENITAL ATROPHY AND ITS SYMPTOMS IN WOMEN**
BEHANDLUNG ODER PRÄVENTION VON UROGENITALER ATROPHIE UND DEREN SYMPTOMEN BEI FRAUEN
TRAITEMENT OU PREVENTION DE L'ATROPHIE UROGÉNITALE ET DE SES SYMPTOMES CHEZ LES FEMMES

(30) Priority: 06.06.2002 US 385904 P
(43) Date of publication of application: 02.03.2005
(73) Proprietor: Hormos Medical Ltd., 20520 Turku (FI)
(72) Inventor: BLOM, Taru, FIN-21270 Nousiainen (FI); GRÖNROOS, Paula, FIN-20540 Turku (FI); HALONEN, Kaija, FIN-21290 Rusko (FI); HÄRKÖNEN, Pirkko, FIN-20720 Turku (FI)
(74) Representative: Öhman, Ann-Marie
(86) International application number: PCT/FI2003/000369
(87) International publication number: WO 2003/103649

(56) References cited:
- WO-A1-02/07718
- US-A- 5 352 699
- US-A- 5 747 059
- US-A1- 2001 034 340

## Description

### FIELD OF THE INVENTION

This invention relates to a method for the inhibition of urogenital atrophy, in women, especially women during or after the menopause. The invention also concerns prevention or treatment of urogenital atrophy-related symptoms in women, especially women during or after menopause.

### BACKGROUND OF THE INVENTION

During and after menopause, elderly women commonly develop symptoms which are due to estrogen deficiency. These symptoms include hot flashes, sweating, insomnia, depression, vaginal dryness, urinary incontinence, nausea, pain, osteoporosis, coronary heart disease, breast tenderness, oedema, fatigue, decreased sexual activity, as well as subsequent psychosocial problems (Payer, 1990; Rekers, 1990). In addition, estrogens are suggested to have neuroprotective effects. Thus, declining estrogen concentrations may negatively affect the mental activities of aging women (Schneider & Finch, 1997; Wickelgren, 1997). Estradiol is known to be excellent in the treatment of climacteric symptoms, and its use in the treatment of these symptoms is rapidly increasing. However, estrogens cause an increased risk of endometrial and breast cancers. It is possible to decrease the carcinogenicity of endometrial cancer by sequential progestin administration, but the risk of breast cancer is not diminished by progestins. The carcinogenicity risk limits the length of estrogen replacement therapy, although it would be very useful to continue the therapy long term, due to the protective effects of estrogens in the bone, in the cardiovascular system, in the central nervous system, and for urinary symptoms.

"SERM"s (selective estrogen receptor modulators) have both estrogen-like and antiestrogenic properties (Kauffman & Bryant, 1995). The effects may be tissue-specific as in the case of tamoxifen and toremifene which have estrogen-like effects in the bone, partial estrogen-like effect in the uterus and liver, and pure antiestrogenic effect in breast cancer. Raloxifene and droloxifen are similar to tamoxifen and toremifene, except that their antiestrogenic properties dominate. Based on the published information, all SERMs are more likely to cause menopausal symptoms than to prevent them. They have, however, other important benefits in elderly women: they decrease total and LDL cholesterol, thus deminishing the risk of cardiovascular diseases, and they may prevent osteoporosis and inhibit breast cancer growth in postmenopausal women. There are also almost pure antiestrogens under development. They are mainly aimed at the treatment of breast cancer (Wakeling & Bowler, 1988).

The compound (deaminohydroxy)toremifene, which also is known under the code FC-1271a or the generic name ospemifene, has relatively weak estrogenic and antiestrogenic effects in the classical hormonal tests (Kangas, 1990). It has antiosteoporosis actions and it decreases total and LDL cholesterol levels in both experimental models and in human volunteers (International patent publications WO 96/07402 and WO 97/32574). It also has antitumor activity in an early stage of breast cancer development in an animal breast cancer model. Ospemifene is the first SERM which has been shown to have beneficial effects in climacteric syndromes in healthy women.

The European patent application EP 664124 A1 suggests the use of raloxifene or related compounds for the inhibition of skin atrophy or vaginal atrophy, especially in postmenopausal women.

WO 02/07718 discloses that that ospemifene, i.e. (deaminohydroxy)toremifene, is effective in treatment of vaginal dryness. However, the document does not teach that ospemifene would be effective against any urinary symptoms or against other vaginal symptoms.

US 5,352,699 discloses a method for treating vaginal atrophy by topical administering of retinoic acid.

US 5,747,059 teaches that 1-centochroman has an advantageous effect on vaginal mucosa in animal tests. There are however essential differences in the structures between compound (I) of the present invention and 1-centochroman: Compound (I) is a triphenylalkene (more specifically triphenylbutene) compound while the compounds disclosed in the cited document are triphenylalkane compounds wherein the alkane chain has created a ring together with a hydroxysubstituent in a phenyl group. Moreover, the compounds disclosed in the cited reference are not said to be SERMs.

US 2001034340 A1 relates to a method for treatment of vaginal atrophy by administering of conjugated estrogens, especially conjugated equine estrogens, USP, and medroxyprogesterone acetate. SERMs are not suggested for this purpose.

### SUMMARY OF THE INVENTION

According to one aspect, this invention concerns the use of the compound of formula (1) 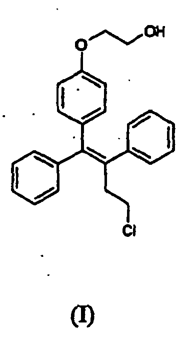 or a geometric isomer, a stereoisomer, a pharmaceutically acceptable salt thereof, or a metabolite thereof selected from the group consisting of TORE VI (4-hydroxy(deaminohydroxy)toremifene), TORE VII (4,4'-dihydroxy-(deaminohydroxy)toremifene), TORE XVIII ((deaminocarboxy)toremifene), TORE VIII (4-hydroxy(deaminocarboxy)toremifene) and TORE XIII (toremifene monophenol), for the manufacture of a pharmaceutical composition for inhibition of urogenital atrophy in women, wherein said urogenital atrophy is related to
i) urinary symptoms selected from the group consisting of micturation disorders, dysuria, hematuria, urinary frequency, sensation of urgency, urinary tract infections, urinary tract inflammation, nocturia, urinary incontinence, urge incontinence and involuntary urinary leakage, or
ii) vaginal symptoms selected from the group consisting of irritation, itching, burning, malodorous discharge, infection, leukorrhea, vulvar pruritus, feeling of pressure and postcoital bleeding.

According to an other aspect, the invention concerns the use of the compound of formula (I) 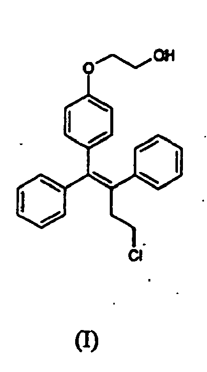 or a geometric isomer, a stereoisomer, a pharmaceutically acceptable salt thereof, or a metabolite thereof selected from the group consisting of TORE VI(4-hydroxy(deaminohydroxy)toremifene), TORE VII (4,4'-dihydroxy-(deaminohydroxy)toremifene), TORE XVIII ((deaminocarboxy)toremifene), TORE VIII (4-hydroxy(deaminocarboxy)toremifene) and TORE XIII (toremifene monophenol), for the manufacture of a pharmaceutical composition for treatment or prevention of symptoms related to urogenital atrophy in women, wherein said symptoms are :
i) urinary symptoms selected from the group consisting of micturation disorders, dysuria, hematuria, urinary frequency, sensation of urgency, urinary tract infections, urinary tract inflammation, nocturia, urinary incontinence, urge incontinence and involuntary urinary leakage, or
ii) vaginal symptoms selected from the group consisting of irritation, itching, burning, malodorous discharge, infection, leukorrhea, vulvar pruritus, feeling of pressure and postcoital bleeding.

### BRIEF DESCRIPTION OF THE DRAWINGS

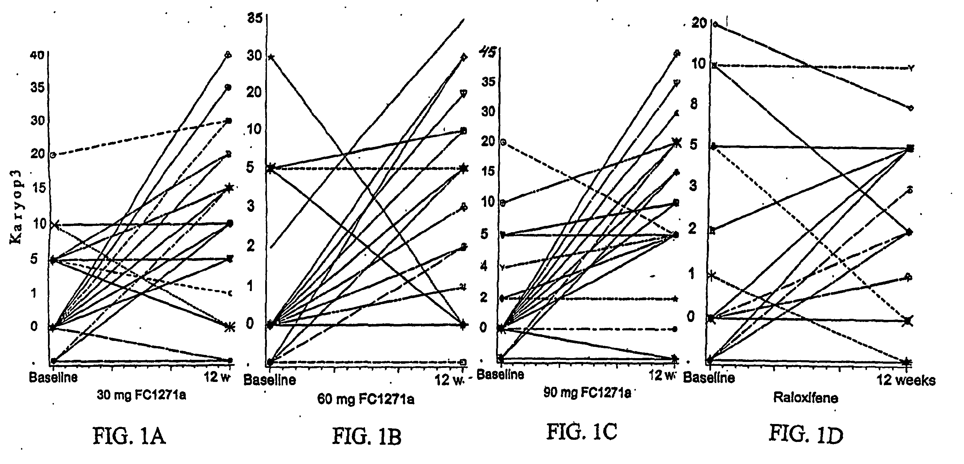

Figures 1A to 1D show changes (from start to 12 weeks' treatment) in the karyopyknosis index for superficial cells of the vaginal epitelium for the individuals treated daily with 30 mg ospemifene, i.e. FC-1271a (1A), 60 mg FC-1271a (1B), 90 mg FC-1271a (1C), and 60 mg raloxifene (1D).

### DETAILED DESCRIPTION OF THE INVENTION

The uses according to this invention are particularly useful for women during or after the menopause. However, the use according to this invention is not restricted to women in this age group.

This invention relates particularly to the use of the estrogen receptor modulator ospemifene in women during or after the menopause. Ospemifene is the Z-isomer of the compound of formula (I) and it is one of the main metabolites of toremifene, is known to be an estrogen agonist and antagonist (Kangas, 1990; International patent publications WO 96/07402 and WO 97/32574).

The term "metabolite" shall be understood to cover the oxidation metabolites mentioned in Kangas (1990) on page 9 (TORE VI, TORE VH, TORE XVIII, TORE VIII, TORE XIII), especially TORE VI and TORE XVIII.

The use of mixtures of isomers of compound (I) shall also be included in this invention.

The atrophy to be inhibited is urogenital atrophy. Symptoms related to urogenital atrophy can be divided in two subgroups: urinary symptoms and vaginal symptoms.

As examples of urinary symptoms can be mentioned micturation disorders, dysuria, hematuria, urinary frequency, sensation of urgency, urinary tract infections, urinary tract inflammation, nocturia, urinary incontinence, urge incontinence and involuntary urinary leakage.

As examples of vaginal symptoms can be mentioned irritation, itching, burning, malodorous discharge, infection, leukorrhea, vulvar pruritus, feeling of pressure and postcoital bleeding.

The effect of atrophy of the skin is cosmetic, but can also be associated with pathological conditions such as decreased ability of the skin to undergo wound healing. Atrophy or aging of skin appears as change of smoothness and texture causing roughness in look and feel on the outer surface of the skin, change of elasticity of the skin effecting the mechanical properties of the skin, and changes in skin pigmentation. Skin aging in postmenopausal women can also be measured as decrease in the mitotic rate of keratinocytes, changes in dermal thickness and decrease in glucosaminoglucans and soluble collagen which are linked to the moisture content of the skin.

The new and surprising effect of ospemifene was found in a clinical study. In this study, raloxifene (60 mg/day) or ospemifene at different doses were given to elderly female volunteers for a period of 3 months. At the dose levels of 30, 60 and 90 mg of ospemifene daily, a significant decrease in vaginal atrophy was observed. These properties are new and unique among the known selective estrogen receptor modulators (SERMs) and indicate that ospemifene at the doses from 25 mg to slightly lower than 100 mg daily, particularly 30 to 90 mg daily, can be successfully used to alleviate symptoms derived from atrophy, especially urogenital atrophy in women during or after the menopause. Furthermore, ospemifene has a superior profile of estrogenic and antiestrogenic effects when compared to any known antiestrogen or SERM compound.

The compound (I) can according to this invention be administered by various routes such as oral, topical, transdermal, intravaginal or subcutaneous routes, of which oral or transdermal administration routes are the most preferable.

Suitable preparation forms include for example tablets, capsules, granules, powders, suspensions, syrups and transdermal formulations, ointments, creams, or gels. Also subcutaneous implants may be useful for prolonged use.

For vaginal local delivery vaginal creams, gels, vagitories, vaginal tablets, pessaries or vaginal rings are preferred.

### EXPERIMENTS

A clinical phase I-II study was carried out to study the effects of ospemifene on endometrial thickness, endometrial pathology, (biopsy taken by curettage as described by Vuopala et al, 1982) and cervical smear in healthy postmenopausal female volunteers in the age range 55 to 69 years. Tolerability and pharmacokinetics were also assessed. Raloxifene (60 mg daily) was used as reference. Ospemifene was given perorally at the doses of 30, 60 and 90 mg daily. There were 29 volunteers at each dose level, as well as in the raloxifene group. Ospemifene was administered in gelatine capsules containing either 30, 60 or 90 mg of ospemifene. The thickness of the endometrium was evaluated by ultrasonography using a Hitachi EUB-405 instrument. The vaginal epithelium was assessed by karyopyknosis index which is a well known assessment method among the skilled persons. In this method, the vaginal fraction of the cervical smears is estimated as the percentage of the number of cells from different layers: the parabasal cell layer; the intermediate cell layer; and the superficial cell layer. Estrogenicity is seen by a shift towards superficial cell fraction. In postmenopausal women this fraction usually is close to zero and estradiol treatment increases the fraction close to 100. Samples were taken before and after the treatment (at 3 months).

### Assessment of the vaginal estrogenic effect of ospemifene (reference example)

Table 1 below shows the change in maturity index for parabasal cells (MI 1) and maturity index for superficial cells (MI 3), after 3 months' administration of varying doses of ospemifene or raloxifene.

**Table 1. Change in maturity index for parabasal cells (MI 1) and maturity index for superficial cells (MI 3), after 3 months' administration of varying doses of ospemifene or raloxifene. (MI 1: index 100 no estrogenicity; index 0 full estrogen, and MI 3: index 100 full estrogen; index 0 no estrogenicity).**

| Compound and dose | MI1 mean | MI1 Sd | MI3 mean | MI3 sd |
|---|---|---|---|---|
| Ospemifene, 30 mg, (n=21) | -40 | 42 | +12.4 | 13.6 |
| Ospemifene, 60 mg, (n=20) | -26 | 39 | +5.5 | 13.4 |
| Ospemifene, 90 mg, (n=22) | -48 | 44 | +12.5 | 14.0 |
| Raloxifene, 60 mg, (n=19) | -2 | 34 | -0.3 | 4.1 |

In Figures 1A to 1D there are shown changes (from start to 12 weeks' treatment) in the karyopyknosis index for superficial cells of the vaginal epithelium for the individuals treated daily with 30 mg ospemifene (1A), 60 mg ospemifene (1B), 90 mg ospemifene (1C), and 60 mg raloxifene (1D). In the Figures, the code FC-1271a is used instead of the generic name ospemifene.

Cervical smear assessments indicate that no one in the raloxifene group (Fig. 1D) had a significant change from baseline to post-treatment in the karyopyknosis index for superficial cells. Most of the individuals in the ospemifene groups had slight increases in the index, but in rest of the subjects the estrogenic effect was very weak, if measurable at all. In all cases the increase was small (< 40 except for one case which was 45 in the 90 mg group) when compared to estradiol which is known to increase the index virtually by 100. A weak but statistically significant estrogenic effect in the cervical smear was therefore documented. No pathological changes were seen in any sample.

### Assessment of the endometrial estrogenic effect of ospemifene (reference example)

Ospemifene had a weak estrogenic effect on endometrial histology. This effect is clearly weaker than that seen with estrogen replacement therapy. There were no malignant findings in the endometrium. The thickness of the endometrium as assessed by ultrasonography showed only a minor, statistically not significant, increase in the thickness (average 0.2 mm, 0.5 mm and 0.5 mm) at the dose levels of 30, 60 and 90 mg, respectively. The measured values were always smaller than 8 mm, which is considered to be a thickness which is indicative for a physiologically significant estrogenicity of SERMs like tamoxifen (Hann et al, 1997; Lahti et al, 1993).

### Effect on urogenital atrophy and symptoms related thereto

In the clinical phase I and II studies, 241 posmenopausal women have been treated with ospemifene. 77 were treated with 25-30 mg, 78 with 50-60 mg, 78 with 90-100 mg and 8 with 200 mg daily dose of ospemifene. In the control groups, 47 were treated with placebo and 29 with raloxifene. Some of the subjects reported spontaneously alleviation of the symptoms associated with urogenital atrophy. The symptoms include both vaginal and urinary symptoms such as vaginal discomfort with irritation, itching, burning, smarting, postcoital bleeding, vulvar itching and/or malodorous discharge and leukorrhea. The urinary symptoms alleviated in individual cases include urinary incontinence, recurrent urinary tract infections, micturition disorders, urinary frequency, nocturia, sensation of urgency, urge incontinence and involuntary urinary leakage. Also, the clinicians reported cases where signs of urogenital atrophy, such as vaginal pallor, petechiae, friability, vaginal mucosa atrophy and ulceration were alleviated by ospemifene.

Based on the present data, the optimal clinical dose is expected to be higher than 25 mg daily and lower than 100 mg daily. A particularly preferable daily dose is found in the range 30 to 90 mg. At the higher doses (100 and 200 mg daily), ospemifene shows properties more similar to those of tamoxifen and toremifene. Ospemifene is an especially valuable drug because it has an excellent tolerability. In addition, ospemifene decreases total and LDL cholesterol, increases HDL cholesterol, and prevents osteoporosis and early stage breast cancer. The present invention suggests that ospemifene and other compounds of formula (I) can be also used during menopause as hormone replacement therapy instead of estrogens, which are known to increase the risk of breast and endometrium cancers.

It will be appreciated that the methods of the present invention can be incorporated in the form of a variety of embodiments, only a few of which are disclosed herein. It will be apparent for the expert skilled in the field that other embodiments exist and do not depart from the spirit of the invention. Thus, the described embodiments are illustrative and should not be construed as restrictive.

### REFERENCES

Delmas PD, Bjarnason NH, Mitlak BH, Ravoux AC, Shah AS, Huster WJ, Draper M, Christiansen C: Effects of raloxifene on bone mineral density, serum cholesterol concentrations, and uterine endometrium in postmenopausal women. N Engl J Med 337: 1641-1647, 1997

Ettinger B, Genant HK, Cann CE: Long-term estrogen replacement therapy prevents bone loss and fractures. Ann Intern Med 102: 319-324, 1985

Hann LE, Giess CS, Bach AM, Tao Y, Baum HJ, Barakat RR: Endometrial thickness in tamoxifen-treated patients: correlation with clinical and pathologic findings. Am J Roentgenol 168: 657-661, 1997

Gustafsson J-Å: Estrogen receptor β- getting in on the action? Nature Medicine 3: 493-494, 1997

Kangas L: Biochemical and pharmacological effects of toremifene metabolites. Cancer Chemother Pharmacol 27: 8-12, 1990

Kauffinan RF, Bryant HU: Selective estrogen receptor modulators. Drug News Perspect 8: 531-539, 1995

Lahti E, Blanco G, Kauppila A, Apaja-Sarkkinen M, Taskinen PJ, Laatikainen T: Endometrial changes in postmenopausal breast cancer patients receiving tamoxifen. Obstet Gynecol 81: 660-664, 1993

Palkowitz AD, Glasebrook AL, Thraser KJ, Hauser KL, Short LL, PhillipsDL, Muchi BS, Sato M, Shetler PK, Cullinan GJ, Pell TR, Bryant HU: Discovery and synthesis of [6-hydroxy-3-[4-[2-(1-piperidinyl)ethoxy]phenoxy]-2-(4- hydroxyphenyl)]benzo[b]thiophene: a novel, highly potent, selective estrogen receptor modulator. Med Chem 40: 1407-1416,1997

Payer L: The menopause in various cultures. In: A portrait of the menopause. Expert reports on medical and therapeutic strategies for the 1990s. Ed. Burger H & Boulet M, Parthenon Publishing, Park Ridge, NJ, USA, 1991. pp 3-22

Rekers H: Matering the menopause. In: A portrait of the menopause. Expert reports on medical and therapeutic strategies for the 1990s. Ed. Burger H & Boulet M, Parthenon Publishing, Park Ridge, NJ, USA, 1991. pp 23-43

Schneider LS, Finch CE: Can estrogens prevent neurodegeneration. Drugs & Aging 11: 87-95, 1997

Spector IP, Carey MP: Incidence and prevalence of sexual dysfunctions: a critical review of the empirical literature. Archives of Sexual Behaviour 19: 389-408, 1990.

Vuopala S, Kauppila A, Mikkonen M, Stenbäck F: Screening of asymptomatic postmenopausal women for gynecological malignancies, with special reference to endometrial sampling methods. Arch Gyncol 231: 119-127, 1982

Wakeling AE, Bowler J: Biology and mode of action of pure antiestrogens. J Steroid Biochem 30: 1-6, 1988

Wickelgren I: Estrogen stakes claim to cognition. Science 276: 675-678, 1997

## Claims

1. Use of the compound of formula (I) 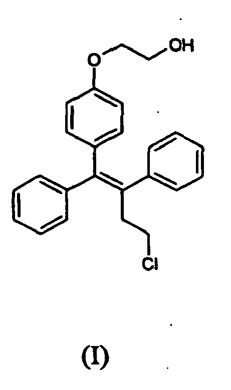 or a geometric isomer, a stereoisomer, a pharmaceutically acceptable salt thereof, or a metabolite thereof selected from the group consisting of TORE VI (4-hydroxy(deaminohydroxy)toremifene), TORE VII (4,4'-dihydroxy-(deaminohydroxy)toremifene), TORE XVIII ((deaminocarboxy)toremifene), TORE VIII (4-hydroxy(deaminocarboxy)toremifene) and TORE XIII (toremifene monophenol), for the manufacture of a pharmaceutical composition for inhibition of urogenital atrophy in women, wherein said urogenital atrophy is related to
i) urinary symptoms selected from the group consisting of micturation disorders, dysuria, hematuria, urinary frequency, sensation of urgency, urinary tract infections, urinary tract inflammation, nocturia, urinary incontinence, urge incontinence and involuntary urinary leakage, or
ii) vaginal symptoms selected from the group consisting of irritation, itching, burning, malodorous discharge, infection, leukorrhea, vulvar pruritus, feeling of pressure and postcoital bleeding.

2. The use according to claim 1 wherein compound (I) is ospemifene.

3. Use of the compound of formula (I) 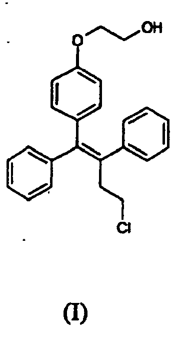 or a geometric isomer, a stereoisomer, a pharmaceutically acceptable salt thereof, or a metabolite thereof selected from the group consisting of TORE VI (4-hydroxy(deaminohydroxy)toremifene), TORE VII (4,4'-dihydroxy-(deaminohydroxy)toremifene), TORE XVIII ((deaminocarboxy)toremifene), TORE VIII (4-hydroxy(deaminocarboxy)toremifene) and TORE XIII (toremifene monophenol), for the manufacture of a pharmaceutical composition for treatment or prevention of symptoms related to urogenital atrophy in women, wherein said symptoms are:
i) urinary symptoms selected from the group consisting of micturation disorders, dysuria, hematuria, urinary frequency, sensation of urgency, urinary tract infections, urinary tract inflammation, nocturia, urinary incontinence, urge incontinence and involuntary urinary leakage, or
ii) vaginal symptoms selected from the group consisting of irritation, itching, burning, malodorous discharge, infection, leukorrhea, vulvar pruritus, feeling of pressure and postcoital bleeding.

4. The use according to claim 3 wherein compound (I) is ospemifene.

5. The use according to any of the foregoing claims wherein the compound is administered orally, topically, transdermally, intravaginally or subcutaneously.

## Patentansprüche

1. Verwendung einer Verbindung der Formel (I) 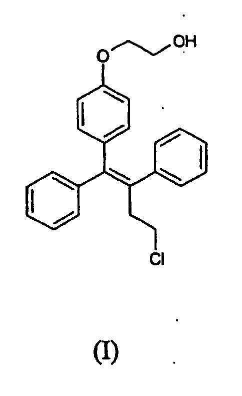 oder eines geometrischen Isomers, eines Stereoisomers, eines pharmazeutisch verträglichen Salzes davon oder eines Metaboliten davon, ausgewählt aus der Gruppe, bestehend aus TORE VI (4-Hydroxy(desaminohydroxy)toremifen), TORE VII (4,4'-Dihydroxy(desaminohydroxy)toremifen), TORE XVIII ((Desaminocarboxy)toremifen), TORE VIII (4-Hydroxy(desaminocarboxy)toremifen) und TORE XIII (Toremifenmonophenol), zur Herstellung einer pharmazeutischen Zusammensetzung zur Hemmung von urogenitaler Atrophie bei Frauen, wobei sicin urogenitale Atrophie bezieht auf
i) Harnsymptome ausgewählt aus der Gruppe bestehend aus Störungen der Mikturation, Dysurie, Hämaturie, Frequenz des Harnlassens, Empfindung der Dringlichkeit, Harnwegeinfektionen, Harnwegeentzündung, Nokturie, Harn-Inkontinenz, Inkontinenz bei Drang und unfreiwilligem Harnabgang oder
ii) vaginalen Symptomen, ausgewählt aus der Gruppe bestehend aus Reizung, Jucken, Brennen, übelriechendem Ausfluss, Infektion, Leukorrhoe, Pruritus der Schamlippen, Gefühl von Druck und postkoitalen Bluten.

2. Verwendung nach Anspruch 1, wobei die Verbindung (I) Ospemifen ist.

3. Verwendung der Verbindung der Formel (I) 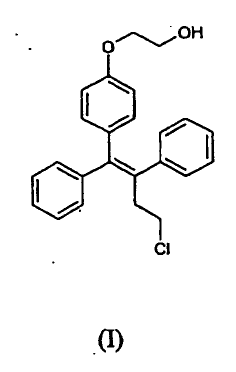 oder eines geometrischen Isomers, eines Stereoisomers, eines pharmazeutisch verträglichen Salzes davon oder eines Metaboliten davon, ausgewählt aus der Gruppe, bestehend aus TORE VI (4-Hydroxy(desaminohydroxy)toremifen), TORE VII (4,4'-Dihydroxy(desaminohydroxy)toremifen), TORE XVIII ((Desaminocarboxy)toremifen), TORE VIII (4-Hydroxy(desaminocarboxy)toremifen) und TORE XIII (Toremifenmonophenol), zur Herstellung einer pharmazeutischen Zusammensetzung zur Behandlung oder Vorbeugung von Symptomen verbunden mit urogenitaler Atrophie bei Frauen, wobei die Symptome sind:
i) Harnsymptome ausgewählt aus der Gruppe bestehend aus Störungen der Mikturation, Dysurie, Hämaturie, Frequenz des Harnlassens, Empfindung der Dringlichkeit, Harnwegeinfektionen, Harnwegeentzündung, Nokturie, Harn-Inkontinenz, Inkontinenz bei Drang und unfreiwilligem Harnabgang oder
ii) vaginalen Symptomen, ausgewählt aus der Gruppe bestehend aus Reizung, Jucken, Brennen, übelriechendem Ausfluss, Infektion, Leukorrhoe, Pruritus der Schamlippen, Gefühl von Druck und postkoitalem Bluten.

4. Verwendung nach Anspruch 3, wobei die Verbindung (I) Ospemifen ist.

5. Verwendung nach einem der voranstehenden Ansprüche, wobei die Verbindung oral, topisch, transdermal, intravaginal oder subkutan verabreicht wird.

## Revendications

1. Utilisation du composé de formule (I) 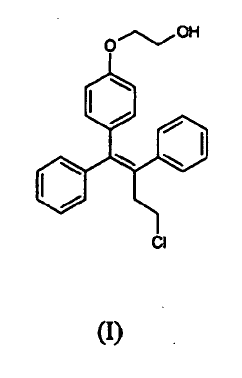 ou d'un isomère géométrique, d'un stéréoisomère, d'un sel acceptable sur le plan pharmaceutique de celui-ci, ou d'un métabolite de ceux-ci choisi dans le groupe constitué par TORE VI (4-hydroxy(déaminohydroxy)-torémifène), TORE VII (4,4'-dihydroxy(déaminohydroxy)-torémifène), TORE XVIII ((déaminocarboxy)torémifène), TORE VIII (4-hydroxy(déaminocarboxy)torémifène) et TORE XIII (monophénol-torémifène) dans la fabrication d'une composition pharmaceutique pour inhiber l'atrophie urogénitale chez la femme, dans laquelle ladite atrophie urogénitale est liée à :
i) des symptômes urinaires choisis dans le groupe constitué par les troubles de la miction, la dysurie, l'hématurie, la pollakiurie, l'impériosité mictionnelle, les infections urinaires, l'inflammation urinaire, la nycturie, l'incontinence urinaire, l'incontinence impérieuse et les fuites urinaires involontaires, ou
ii) des symptômes vaginaux choisis dans le groupe constitué par les irritations, les démangeaisons, les brûlures, les pertes vaginales malodorantes, les infections, la leucorrhée, le prurit vulvaire, la sensation de pression et les saignements post-coïtaux.

2. Utilisation selon la revendication 1, dans laquelle le composé (I) est l'ospémifène.

3. Utilisation du composé de formule (I) 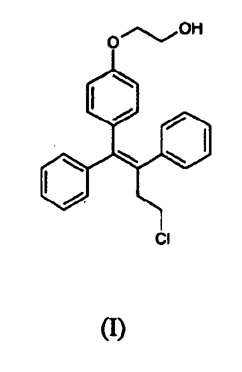 ou d'un isomère géométrique, d'un stéréoisomère, d'un sel acceptable sur le plan pharmaceutique de celui-ci, ou d'un métabolite de ceux-ci choisi dans le groupe constitué par TORE VI (4-hydroxy(déaminohydroxy)-torémifène), TORE VII (4,4'-dihydroxy(déaminohydroxy)-torémifène), TORE XVIII ((déaminocarboxy)torémifène), TORE VIII (4-hydroxy(déaminocarboxy)torémifène) et TORE XIII (monophénol-torémifène) dans la fabrication d'une composition pharmaceutique pour le traitement ou la prévention de symptômes liés à l'atrophie urogénitale chez la femme, dans laquelle lesdits symptômes sont :
i) des symptômes urinaires choisis dans le groupe constitué par les troubles de la miction, la dysurie, l'hématurie, la pollakiurie, l'impériosité mictionnelle, les infections urinaires, l'inflammation urinaire, la nycturie, l'incontinence urinaire, l'incontinence impérieuse et les fuites urinaires involontaires, ou
ii) des symptômes vaginaux choisis dans le groupe constitué par les irritations, les démangeaisons, les brûlures, les pertes vaginales malodorantes, les infections, la leucorrhée, le prurit vulvaire, la sensation de pression et l'es saignements post-coïtaux.

4. Utilisation selon la revendication 3, dans laquelle le composé (I) est l'ospémifène.

5. Utilisation selon l'une quelconque des revendications précédentes, dans laquelle le composé est administré par voie orale, topique, transdermique, intra-vaginale ou sous-cutanée.
